# FASCICULE DE BREVET EUROPEEN

(11) **EP 1 228 484 B1**
(45) Date de publication et mention de la délivrance du brevet: **21.05.2003**
(21) Numéro de dépôt: 00968024.0
(22) Date de dépôt: 12.10.2000
(51) Int. Cl.: G06M 1/24

(54) **COMPTEUR DE DOSES ET DISTRIBUTEUR DE PRODUIT FLUIDE INCORPORANT UN TEL COMPTEUR**
DOSENZÄHLER UND SPENDER FÜR FLÜSSIGE MEDIEN DIE EIN SOLCHER ZÄHLER UMFASST
DOSE COUNTER AND DISTRIBUTOR OF A FLUID PRODUCT INCORPORATING A COUNTER OF THIS TYPE

(30) Priorité: 19.10.1999 FR 9913001
(43) Date de publication de la demande: 07.08.2002
(73) Titulaire: VALOIS S.A.S., 27110 Le Neubourg (FR)
(72) Inventeur: STRADELLA, Giuseppe, I-16032 Camogli (IT)
(74) Mandataire: CAPRI SARL
(86) Numéro de dépôt international: FR0002845
(87) Numéro de publication internationale: WO01029765

(56) Documents cités:
- CH-A- 547 537
- DE-C- 815 563
- FR-A- 2 750 780

## Description

La présente invention concerne un dispositif de compteur de doses et un distributeur de produit fluide incorporant un tel compteur de doses.

Les systèmes de compteurs de doses pour des dispositifs de distribution de produit fluide sont bien connus dans l'état de la technique. Ils comportent généralement des moyens de comptage réalisés le plus souvent sous la forme de disques rotatifs, qui sont tournés d'un angle prédéterminé à chaque actionnement du distributeur de produit fluide. Ces disques comportent généralement des chiffres pour indiquer le nombre de doses distribuées ou restant à distribuer, ces chiffres étant prévus le plus souvent sur le bord périphérique extérieur des disques de comptage, c'est-à-dire sur la tranche externe définissant l'épaisseur dudit disque de comptage. Ainsi, à chaque actionnement du distributeur de produit fluide, un chiffre correspondant passe devant une fenêtre ménagée de manière appropriée, permettant de visualiser ledit chiffre depuis l'extérieur de l'appareil. Des compteurs de ce type sont notamment divulgués dans les documents EP-0 484 188, EP-0 472 915, EP-0 539 469, EP-0 764 312 et WO 98/01822.

Ces compteurs présentent un certain nombre d'inconvénients. D'une part, leur présence dans un appareil de distribution de produit fluide implique une augmentation de la dimension dudit appareil, la place que prend un système de compteur étant d'autant plus grande que le nombre de doses à compter est important. En particulier, pour pouvoir compter un nombre de doses important, il faut soit augmenter le diamètre du disque de comptage pour pouvoir inscrire plus de chiffres sur la tranche de celui-ci, soit prévoir deux ou plusieurs disques de comptage superposés coopérant par exemple au moyen d'un système de came, les disques représentant alors respectivement les unités, les dizaines, les centaines, etc. La dimension du système de compteur est dans ce cas fortement augmenté dans la direction de l'axe de rotation desdits disques de comptage. D'autre part, de tels systèmes de compteur de doses ne sont pas facilement adaptables dans un dispositif de distribution, et des moyens d'actionnement du compteur relativement complexes sont généralement nécessaires pour coupler l'actionnement du compteur à celui du dispositif de distribution de produit fluide. De plus, ce type de compteur n'est généralement pas adaptable à des dispositifs de distribution comportant des courses d'actionnement variables. Ainsi, par exemple, dans le cas d'une valve doseuse, la course d'actionnement de la soupape peut varier selon le modèle de valve doseuse utilisé, de sorte que des moyens d'actionnement spécifiques doivent être prévus pour chaque modèle de valve. D'autre part, la course d'actionnement de la soupape d'une valve doseuse peut varier entre deux actionnements successifs de ladite valve, ce qui peut entraîner un dysfonctionnement dans le comptage des doses, une dose pouvant ne pas être comptabilisée ou au contraire comptée deux fois en raison d'une course d'actionnement variable trop faible resp. trop grande.

La présente invention a pour but de fournir un dispositif de compteur de doses pour un distributeur de produit fluide qui ne reproduit pas les inconvénients susmentionnés.

En particulier, la présente invention a pour but de fournir un dispositif de compteur de doses qui soit simple et peu coûteux à fabriquer et à assembler.

La présente invention a également pour but de fournir un tel dispositif de compteur de doses qui ne modifie substantiellement pas la dimension extérieure du dispositif de distribution de produit fluide sur lequel il est appliqué.

La présente invention a également pour but de fournir un tel dispositif de compteur de doses qui est capable de compter un nombre quelconque de doses, y compris des nombres très élevés, sans substantiellement modifier la dimension du distributeur de produit fluide.

La présente invention a encore pour but de fournir un tel dispositif de compteur de doses qui soit utilisable avec des organes d'actionnement, notamment des valves, ayant des courses d'actionnement variables.

La présente invention a donc pour objet un dispositif de compteur de doses pour distributeur de produit fluide, comportant des moyens de comptage actionnés par des moyens d'actionnement lors de chaque utilisation du distributeur, caractérisée en ce que lesdits moyens de comptage comportant au moins un élément de comptage rotatif flexible appliqué contre et tournant sur une surface de support courbe.

De préférence, ledit élément de comptage flexible se déforme à chaque actionnement pour rester en contact avec ladite surface de support courbe.

Avantageusement, ledit élément de comptage flexible est en forme de disque et comporte une dentelure annulaire ou similaire sur son bord externe, adaptée à coopérer avec lesdits moyens d'actionnement.

Selon un mode de réalisation particulier de la présente invention, lesdits moyens de comptage comportent deux éléments de comptage rotatifs flexibles superposés, lesdits moyens d'actionnement coopérant avec le premier élément de comptage et des moyens de couplage étant prévus entre les deux éléments de comptage pour entraîner en rotation le second élément de comptage, lesdits moyens de couplage étant déplaçables entre une position de non-couplage et une position de couplage.

Avantageusement, lesdits éléments de comptage tournent autour d'un axe de rotation commun, lesdits moyens de couplage comportant une languette élastique solidaire de l'un des deux éléments de comptage coopérant avec une fente prévue dans l'autre élément de comptage, ladite languette étant sollicitée dans la position de couplage à chaque tour complet du premier élément de comptage par des moyens de came.

En variante, lesdits éléments de comptage tournent autour d'axes de rotation parallèles décalés, lesdits moyens de couplage comportant un ergot solidaire de l'un des deux éléments de comptage, coopérant avec une projection solidaire de l'autre élément de comptage et parvenant en position de couplage à chaque tour complet du premier élément de comptage.

Avantageusement, le second élément de comptage est réalisé sous la forme d'un disque flexible ou d'une section angulaire de disque flexible.

Avantageusement, l'un des deux éléments de comptage flexibles superposés comporte une fenêtre pour visualiser partiellement l'autre élément de comptage.

Avantageusement, l'un des deux éléments de comptage flexible superposés est réalisé de manière transparente pour visualiser l'autre élément de comptage.

La présente invention a également pour objet un distributeur de produit fluide comportant un réservoir de produit, un organe de distribution, tel qu'une pompe ou une valve, et un orifice de distribution, pour distribuer sélectivement ledit produit, ledit distributeur comportant un disque de compteur de doses.

De préférence, ledit au moins un élément de comptage est disposé entre deux surfaces environ cylindrique concentriques, l'axe de rotation dudit au moins un élément de comptage étant environ perpendiculaire auxdites surfaces environ cylindriques.

Avantageusement, ledit au moins un élément de comptage est disposé entre le corps externe du distributeur et un corps interne à l'intérieur duquel est monté le réservoir.

Selon un premier mode de réalisation de l'invention, ledit réservoir est monté coulissant à l'intérieur dudit corps interne, ledit réservoir coopérant avec lesdits moyens d'actionnement du dispositif de compteur de doses, de sorte que le déplacement du réservoir entraîne l'actionnement du compteur.

Avantageusement, lesdits moyens d'actionnement comportent un doigt d'actionnement déplaçable ensemble avec le réservoir pour coopérer avec ledit élément de comptage, ledit doigt étant flexible au moins dans la direction de déplacement du réservoir, de telle sorte qu'il peut s'adapter à des courses de déplacement du réservoir variables.

Avantageusement, l'organe de distribution est une valve doseuse adaptée à distribuer un produit avec un gaz propulseur, la course de déplacement du réservoir correspondante à la course d'actionnement de la soupape de la valve doseuse.

Avantageusement, ledit doigt flexible fait partie d'un organe actionneur monté coulissant entre ledit corps interne du distributeur et ledit réservoir, ledit corps interne définissant une fenêtre à travers laquelle ledit doigt flexible coopère avec ledit élément de comptage, ladite fenêtre comportant une butée de déplacement axial pour ledit doigt flexible, de sorte qu'un déplacement axial supplémentaire entraîne une déformation élastique dudit doigt flexible.

Selon un second mode de réalisation de la présente invention, le réservoir est monté fixement dans le corps du distributeur, ledit distributeur comportant un élément de distribution pour actionner ledit organe de distribution, lesdits moyens d'actionnement dudit dispositif compteur de doses étant solidaire dudit élément de distribution.

Avantageusement, ledit élément de distribution est un capot pivotant entre une position de fermeture de l'orifice de distribution et une position de distribution, l'organe de distribution étant une pompe déclenchable par l'inhalation.

D'autres caractéristiques et avantages de la présente invention apparaîtront au cours de la description détaillée suivante de deux modes de réalisation particuliers de la présente invention, donnés à titre d'exemples non limitatifs en référence aux dessins joints, sur lesquels :
- la figure 1 est une vue schématique en section partiellement découpée d'un distributeur de produit fluide incorporant un dispositif de compteur de doses selon la présente invention,
- la figure 2 est une vue schématique en section horizontale le long de la ligne I-I du dispositif représenté sur la figure 1,
- la figure 3 est une vue schématique en section transversale partiellement découpée d'un distributeur de produit fluide incorporant un dispositif de compteur de doses selon un second mode de réalisation de la présente invention,
- la figure 4 est une vue similaire à celle de la figure 3, en positon d'actionnement du distributeur de produit fluide,
- la figure 5 est une vue schématique en section horizontale le long de la ligne I-I du dispositif de la figure 4,
- les figures 6 et 7 sont des vues schématiques d'un dispositif de compteur de doses comportant deux éléments de comptage superposés, selon une première variante de réalisation de la présente invention, et
- les figures 8 et 9 sont des vues schématiques d'un dispositif de compteur de doses comportant deux éléments de comptage superposés, selon une seconde variante de réalisation de la présente invention.

Le distributeur de compteur de doses selon la présente invention comporte un ou plusieurs éléments de comptage rotatifs flexibles. Avantageusement, cet élément de comptage est réalisé sous la forme d'un disque souple ou flexible 10 qui est appliqué contre une surface de support courbe, notamment environ cylindrique. Le disque flexible 10 peut être appliqué contre une surface convexe ou contre une surface concave selon qu'il est placé à l'intérieur ou à l'extérieur de ladite surface de support. Toutefois, dans la description ci-après, et dans les modes de réalisation préférés, le disque de comptage 10 est avantageusement disposé entre deux surfaces concentriques qui définissent entre elles un petit écart dans lequel est disposé ledit disque souple 10. Avantageusement, ces deux surfaces sont constituées par le corps externe 1 du dispositif de distribution de produit fluide et un corps interne 2 fixé audit corps externe 1.

Le ou les éléments de comptage 10 tournent avantageusement autour d'un axe de rotation environ perpendiculaire auxdits ou de ladite surface(s) courbe(s) de support. Comme cela sera décrit plus en détail ci-après, lorsque deux disques ou plus sont prévus, ceux-ci peuvent tourner autour d'un axe de rotation commun 60 ou autour de deux axes de rotation décalés 60, 61.

Le disque de comptage flexible 10 coopère avec de moyens d'actionnement 20, et le disque de comptage 10 comporte avantageusement sur son bord périphérique externe une dentelure annulaire 15, ou similaire. Les moyens d'actionnement 20 viennent successivement en prise avec une dent respective de ladite dentelure annulaire 15, à chaque actionnement dudit dispositif de distribution de produit fluide. Avantageusement, lesdits moyens d'actionnement comprennent un doigt d'actionnement 20. Ce doigt d'actionnement 20 est de préférence flexible, comme cela sera décrit plus en détail ci-après. On peut également prévoir des moyens anti-retour 40 pour empêcher une rotation du ou des disques de comptage dans une direction opposée à celle imprimée par lesdits moyens d'actionnement 20. Ces moyens anti-retour peuvent comprendre une patte flexible 40 coopérant avec ladite dentelure annulaire 15, d'une manière connue.

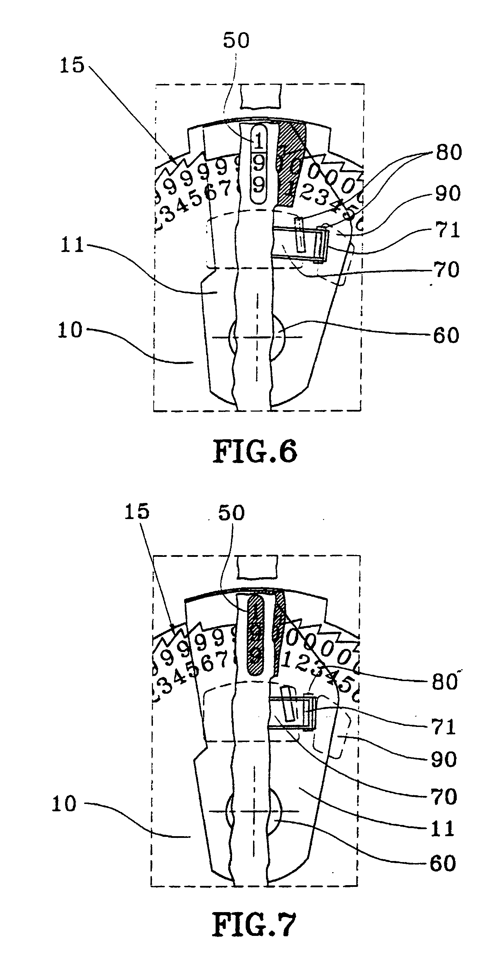

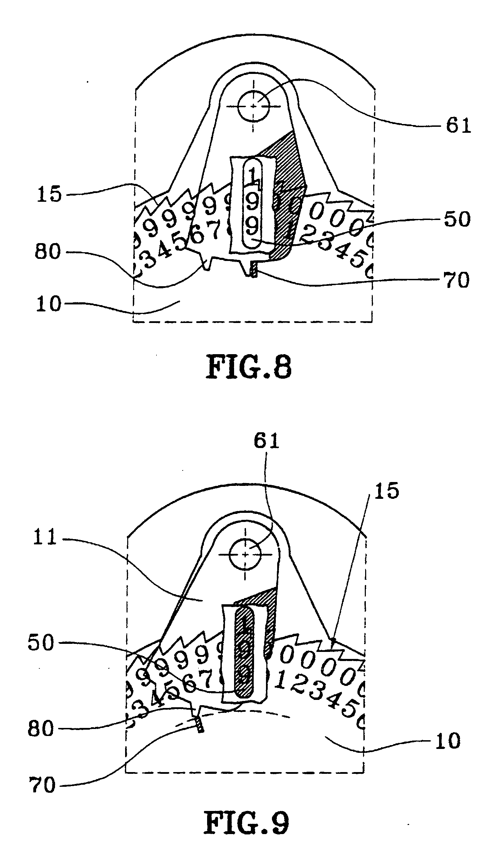

En référence aux figures 6 à 9, il est représenté deux variantes de réalisation d'un dispositif de compteur de doses comportant deux éléments de comptage superposés 10 et 11. Le premier mode de réalisation, représenté sur les figures 6 et 7, montre un premier élément de comptage 10 réalisé sous la forme d'un disque souple comportant sur son bord périphérique externe une dentelure annulaire 15 coopérant avec des moyens d'actionnement 20 (non représentés sur ces figures). Chaque dent de la dentelure annulaire 15 correspond à un chiffre qui, dans l'exemple représenté, vont de 0 à 99, c'est-à-dire que la dentelure annulaire 15 comporte 100 dents. Ledit premier disque souple 10 tourne autour d'un axe perpendiculaire à l'axe longitudinal central de la surface courbe sur laquelle est appliqué ledit disque de comptage souple 10. Un second élément de comptage rotatif souple 11 est superposé au premier disque de comptage 10, et est dans l'exemple représenté constitué d'un secteur de disque. Mais il pourrait également être réalisé sous la forme d'un disque complet en fonction de l'échelle d'indication souhaitée. Les deux éléments de comptage, à savoir le premier disque flexible 10 et le secteur de disque flexible superposé 11 tournent autour du même axe de rotation 60, et des moyens de couplage 70, 80 sont prévus entre les deux éléments de comptage 10 et 11 pour coupler de manière prédéterminée ces deux éléments de comptage, de sorte qu'une rotation du premier disque de comptage souple 10 entraîne la rotation simultanée du secteur de disque souple 11 superposé. Ces moyens de couplage peuvent avantageusement comporter une languette flexible 70 pourvue à son extrémité d'une projection 71 formant une sorte de crochet. Le second élément de comptage, en l'occurrence le secteur de disque 11, est lui pourvu d'une ou plusieurs fentes 80 positionnées de telle manière à recevoir ladite projection 71 lorsque les deux disques de comptage doivent être couplés. Des moyens de came 90 sont prévus pour solliciter la languette flexible 70 et la déformer de telle sorte que la projection 71 pénètre dans la fente, ce qui solidarise les deux éléments de comptage 10 et 11. Ce couplage est de préférence réalisé à chaque tour complet du premier disque de comptage 10. Lesdits moyens de came 90 peuvent être formés notamment par un bossage ou similaire prévus dans la surface de support sur laquelle est appliqué le premier disque de comptage 10. Bien entendu, tous moyens équivalent permettant de solliciter la languette flexible 70 du premier disque de comptage 10 dans la fente 80 du second disque de comptage 11 sont envisageables. De même, lesdits moyens de couplage 70 et 80 peuvent être réalisés d'une manière différente. En particulier, ces moyens de couplage pourraient être inversés, à savoir que c'est le second élément de comptage 11 qui supporte le moyen de couplage élastique 70 et le premier disque de couplage 10 qui incorpore la ou les fentes 80.

En référence aux figues 8 et 9, il est représenté une deuxième variante de réalisation. dans lequel les deux éléments de comptage rotatif tournent respectivement autour d'axes de rotation différents 60 et 61. A nouveau dans cet exemple, le second élément de comptage 11 est réalisé sous la forme d'un secteur de disque, mais il pourrait aussi être réalisé sous la forme d'un disque complet, si souhaité. Dans cet exemple, les moyens de couplage comportent un ergot 70 solidaire du premier élément de comptage qui est adapté à coopérer avec des projections 80 prévues sur le second élément de comptage 11. A chaque tour complet du premier élément de comptage, ledit ergot 70 vient entraîner une projection 80 du second élément de comptage 11, couplant par là lesdits deux éléments de comptage. Tout autre variante des moyens de couplage est également envisageable ici.

Dans les deux exemples de réalisation décrits ci-dessus, en référence aux figures 6 à 9, le second élément de comptage 11 est avantageusement disposé sur le premier élément de comptage 10, dans la direction de visualisation par l'utilisateur, c'est-à-dire que le second élément de comptage 11 est interposé entre le premier élément de comptage 10 et une fenêtre de visualisation 50 prévue dans le corps externe 1 du dispositif de distribution de produit fluide. Dans ce cas, les numéros ou indications sur le second élément de comptage sont avantageusement imprimés sur une surface transparente, de sorte que ce second élément de comptage 11 ne cache pas les numéros ou indications sur le premier élément de comptage 10, disposé en dessous. Eventuellement, on pourrait également prévoir une fenêtre de visualisation permettant de voir les chiffres inscrits sur l'élément de comptage 10 disposé en dessous.

De manière avantageuse, le second élément de comptage 11 comporte une partie obscurcie qui vient se mettre en regard de la fenêtre de la visualisation 50 de l'appareil de distribution de produit fluide après distribution de la dernière dose de produit. Ceci permet de prédéterminer un nombre théorique de doses maximales à distribuer par l'appareil de produit fluide, de sorte qu'après la distribution de la dernière dose théorique, la fenêtre de visualisation est obscurcie et l'utilisateur sait que l'appareil ne devrait plus être utilisé. Par contre, l'actionnement de l'appareil n'est pas bloqué ou empêché, et des actionnements supplémentaires de l'appareil de distribution de produit fluide sont possibles, sans qu'aucune indication ne soit affichée dans la fenêtre de visualisation 50.

Le dispositif de compteur de doses de la présente invention s'applique à tout type de dispositif de distribution de produit fluide, et la description détaillée suivante sera faite en référence à deux modes de réalisation particuliers, où le dispositif de compteur de doses est appliqué d'une part dans un appareil du type inhalateur actionné par l'inhalation (BAI), et d'autre part dans un appareil du typé inhalateur d'aérosol (MDI). Il est entendu que la présente invention ne se limite pas à ces deux exemples.

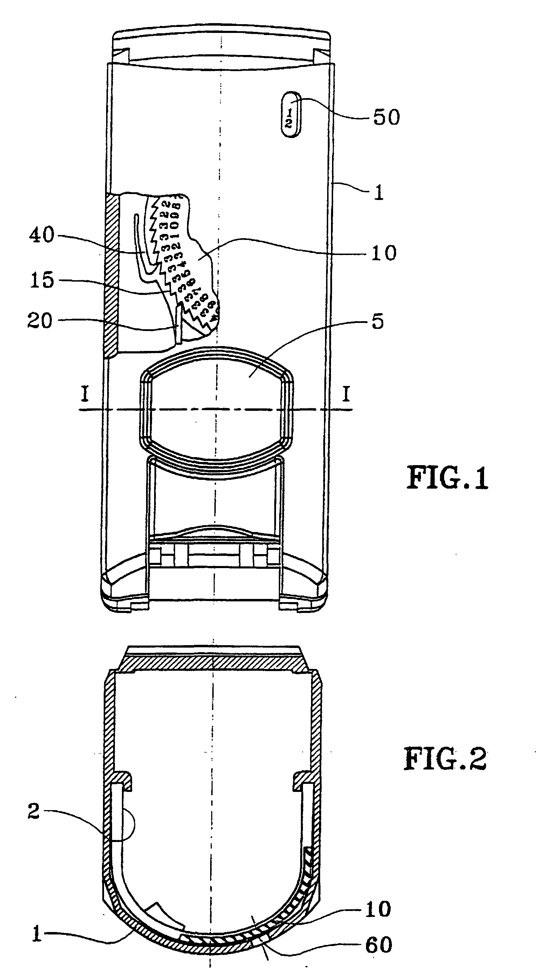

En référence aux figures 1 et 2, il est représenté un inhalateur, en particulier un inhalateur déclenché par l'inhalation du patient. Le dispositif comporte un corps externe 1 pourvu d'une fenêtre de visualisation 50. L'élément de comptage 10 est appliqué à l'intérieur dudit corps externe 1 contre un corps interne 2 formant surface de support courbe. Comme représenté sur la figure 2, la surface courbe n'est pas nécessairement cylindrique, le caractère flexible de l'élément de comptage 10 lui permettant une rotation autour de son axe de rotation 60 en restant en permanence en contact des deux surfaces courbes qui l'entourent, à savoir le corps externe et le corps interne 1 et 2 du distributeur. indépendamment de la forme desdites surfaces courbes. Les moyens d'actionnement comportent un doigt d'actionnement 20 déplaçable axialement et dont l'extrémité coopère avec la dentelure périphérique de l'élément de comptage 10. Avantageusement, ledit doigt d'actionnement 20 est solidaire d'un poussoir, ou d'autre élément de distribution de ce type. En particulier, le doigt d'actionnement 20 peut être solidaire d'un capot mobile entre une position de fermeture de l'orifice de distribution 5 et une position de distribution, le capot étant monté pivotant sur le corps externe du distributeur, le pivotement dudit capot provoquant la course axiale du doigt d'actionnement 20 et donc la rotation de l'élément de comptage 10.

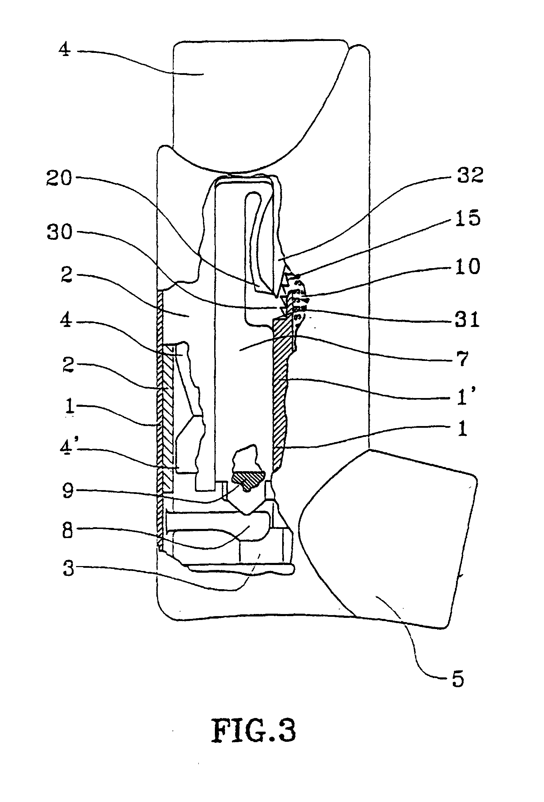

En référence aux figures 3 à 5, il est représenté un second mode de réalisation, appliqué à un inhalateur aérosol, communément appelé MDI. Ce type d'appareil comporte généralement un corps externe 1 incorporant un embout buccal contenant l'orifice de distribution 5. Le réservoir de produit 4, contenant le produit dispersé dans un gaz propulseur, est monté coulissant à l'intérieur dudit corps externe 1 et est pourvu d'une valve doseuse. Le déplacement du réservoir 4 entraîne donc le déplacement de la soupape de la valve doseuse 3 (non représentée en détail), et par là l'expulsion d'une dose de produit. Un corps interne 2 est monté à l'intérieur du corps externe 1, l'élément de comptage 10 étant prévu entre les corps interne 2 et externe 1. Avantageusement, le réservoir 4 est cylindrique de même que les corps interne et externe, de sorte que l'élément de comptage 10 est appliqué contre une surface cylindrique. La présente invention s'applique toutefois également à des surfaces courbes de support non cylindriques. Un organe actionneur 7 est monté coulissant entre le corps interne 2 et le réservoir 4, ledit organe actionneur 7 incorporant un doigt flexible d'actionnement 20 dont l'extrémité est destinée à coopérer avec la dentelure périphérique 15 de l'élément de comptage 10. L'organe actionneur 7 est déplaçable axialement contre un élément élastique 8, tel qu'une patte flexible ou un ressort, adapté à ramener ledit organe actionneur 7 en position de départ après un cycle d'actionnement complet. L'élément actionneur 7 comprend également avantageusement un épaulement de commande 9 qui s'appuie sur le col 4' du réservoir 4 contenant le produit. L'actionneur 7 qui coulisse à l'intérieur du corps interne 2 est avantageusement monté coulissant sur un siège de support formé partiellement par le corps interne 2 et complété par une nervure 1' formée dans le corps externe 1. Une paroi latérale 32 dudit siège et ladite nervure 1' définissent une ouverture ou une fenêtre 30 à travers laquelle l'extrémité du doigt flexible 20 peut pénétrer pour coopérer avec l'organe de comptage 10. Avantageusement, la nervure 1' est pourvue d'un épaulement 31 qui forme une butée de déplacement axial pour le doigt d'actionnement flexible 20.

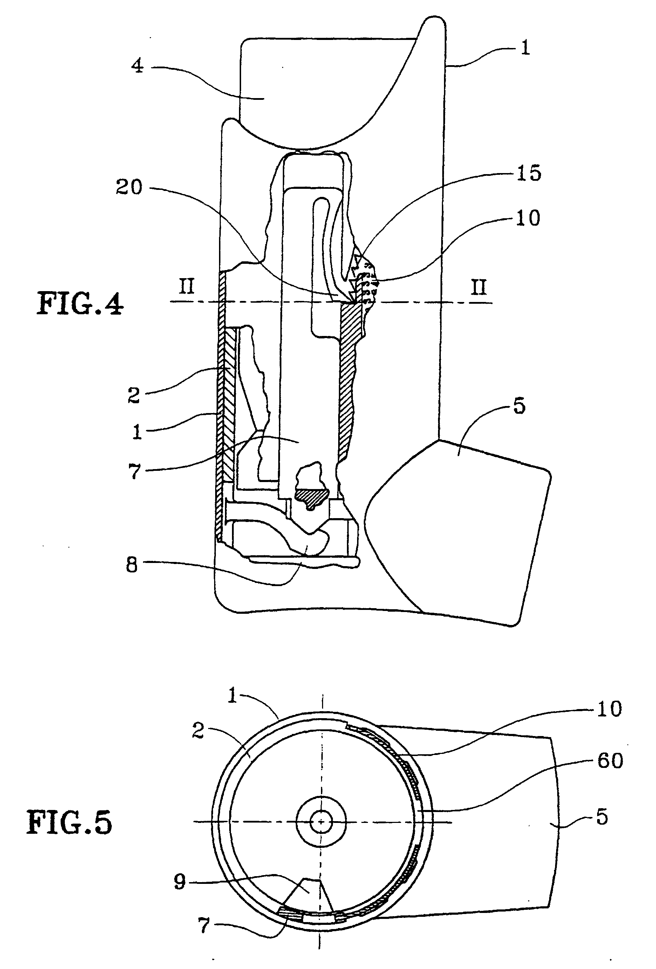

Le fonctionnement du système représenté sur les figures 3 à 5 est le suivant. Lors de l'actionnement du dispositif de distribution de produit fluide, le réservoir 4 est déplacé verticalement vers le bas, en se référant à la représentation sur les figures 3 et 4. Sous l'effet de l'épaulement de commande 9, l'organe actionneur 7 coulisse ensemble avec ledit réservoir 4. Ainsi, le bout du doigt d'actionnement élastique 20 entre dans la fenêtre 30 et coopère avec une dent de l'élément de comptage flexible 10, entraînant sa rotation. L'angle de rotation de l'élément de comptage 10 est déterminé et limité par la position de l'épaulement 31, de sorte que cet angle de rotation est indépendant de la longueur de la course d'actionnement du réservoir 4. En effet, si la course d'actionnement du réservoir 4 est supérieure, le bout du doigt flexible 20 est arrêté par l'épaulement de butée 31, et tout déplacement axial supplémentaire est absorbé par la flexion dudit doigt flexible 20. Avantageusement, ce doigt est flexible à la fois horizontalement et verticalement. De manière similaire, pendant le retour de l'organe actionneur 7 vers sa position de repos, sous l'effet de l'élément élastique 8, le bout du doigt d'actionnement 20 sort de la fenêtre 30 toujours dans la même position du fait de la présence de la paroi latérale 32 et tout mouvement vertical supplémentaire est absorbé par une flexion horizontale dudit doigt flexible 20. La course d'actionnement dudit réservoir 4 correspondant à la course d'actionnement de la valve doseuse 3 montée sur ledit réservoir, le système de compteur de la présente invention s'applique donc à des valves différentes ayant des courses d'actionnement différentes. Il permet également de compter de manière sure les doses distribuées par une valve doseuse ayant des courses d'actionnement variables entre deux actionnements successifs.

Selon le positionnement de la fenêtre 30 défini entre l'épaulement 31 de la nervure 1' et la paroi latérale 32 du siège de l'organe actionneur 7, la rotation de l'élément de comptage 10 est réalisée au début de la course d'actionnement du réservoir 4, à la fin de celle-ci ou entre les deux.

Bien entendu, diverses modifications sont envisageables sans sortir du cadre de la présente invention dont la portée est définie par les revendications annexées.

## Revendications

1. Dispositif de compteur de doses pour distributeur de produit fluide, comportant des moyens de comptage (10, 11) actionnés par des moyens d'actionnement (20) lors de chaque utilisation du distributeur, **caractérisée en ce que** lesdits moyens de comptage comportent au moins un élément de comptage rotatif flexible (10) appliqué contre et tournant sur une surface de support courbe (1, 2).

2. Dispositif selon la revendication 1, dans lequel ledit élément de comptage flexible (10) se déforme à chaque actionnement pour rester en contact avec ladite surface de support courbe (1, 2).

3. Dispositif selon la revendication 1 ou 2, dans lequel ledit élément de comptage flexible (10) est en forme de disque et comporte une dentelure annulaire (15) ou similaire sur son bord externe, adaptée à coopérer avec lesdits moyens d'actionnement (20).

4. Dispositif selon l'une quelconque des revendications précédentes, dans lequel lesdits moyens de comptage comportent deux éléments de comptage rotatifs flexibles superposés (10, 11), lesdits moyens d'actionnement (20) coopérant avec le premier élément de comptage (10) et des moyens de couplage (70, 80) étant prévus entre les deux éléments de comptage (10, 11) pour entraîner en rotation le second élément de comptage (11), lesdits moyens de couplage (70, 80) étant déplaçables entre une position de non-couplage et une position de couplage.

5. Dispositif selon la revendication 4, dans lequel lesdits éléments de comptage (10, 11) tournent autour d'un axe de rotation commun (60), lesdits moyens de couplage comportant une languette élastique (70) solidaire de l'un des deux éléments de comptage (10, 11) coopérant avec une fente (80) prévue dans l'autre élément de comptage (11, 10), ladite languette (70) étant sollicitée dans la position de couplage à chaque tour complet du premier élément de comptage (10) par des moyens de came (90).

6. Dispositif selon la revendication 4, dans lequel lesdits éléments de comptage (10, 11) tournent autour d'axes de rotation parallèles décalés (60, 61), lesdits moyens de couplage comportant un ergot (70) solidaire de l'un des deux éléments de comptage (10, 11), coopérant avec une projection (80) solidaire de l'autre élément de comptage (11, 10) et parvenant en position de couplage à chaque tour complet du premier élément de comptage (10).

7. Dispositif selon l'une quelconque des revendications 4 à 6, dans lequel le second élément de comptage (11) est réalisé sous la forme d'un disque flexible ou d'une section angulaire de disque flexible.

8. Dispositif selon l'une quelconque des revendications 4 à 7, dans lequel l'un des deux éléments de comptage flexibles superposés (10, 11) comporte une fenêtre (100) pour visualiser partiellement l'autre élément de comptage (11, 10).

9. Dispositif selon l'une quelconque des revendications 4 à 8, dans lequel l'un des deux éléments de comptage flexible superposés (10, 11) est réalisé de manière transparente pour visualiser l'autre élément de comptage (11, 10).

10. Distributeur de produit fluide comportant un réservoir de produit, un organe de distribution (3), tel qu'une pompe ou une valve, et un orifice de distribution (5), pour distribuer sélectivement ledit produit, **caractérisé en ce que** ledit distributeur comporte un dispositif de compteur de doses selon l'une quelconque des revendications 1 à 9.

11. Distributeur selon la revendication 10, dans lequel ledit au moins un élément de comptage (10) est disposé entre deux surfaces (1, 2) environ cylindrique concentriques, l'axe de rotation (60) dudit au moins un élément de comptage (10) étant environ perpendiculaire auxdites surfaces environ cylindriques (1, 2).

12. Distributeur selon la revendication 10 ou 11, dans lequel ledit au moins un élément de comptage (10) est disposé entre le corps externe (1) du distributeur et un corps interne (2) à l'intérieur duquel est monté le réservoir (4).

13. Distributeur selon la revendication 12, dans lequel ledit réservoir (4) est monté coulissant à l'intérieur dudit corps interne (2), ledit réservoir (4) coopérant avec lesdits moyens d'actionnement (20) du dispositif de compteur de doses, de sorte que le déplacement du réservoir (4) entraîne l'actionnement du compteur.

14. Distributeur selon la revendication 13, dans lequel lesdits moyens d'actionnement (20) comportent un doigt d'actionnement (20) déplaçable ensemble avec le réservoir (4) pour coopérer avec ledit élément de comptage (10), ledit doigt (20) étant flexible au moins dans la direction de déplacement du réservoir (4), de telle sorte qu'il peut s'adapter à des courses de déplacement variables du réservoir.

15. Distributeur selon la revendication 14, dans lequel l'organe de distribution (3) est une valve doseuse adaptée à distribuer un produit avec un gaz propulseur, la course de déplacement du réservoir (4) correspondant à la course d'actionnement de la soupape de la valve doseuse (3).

16. Distributeur selon la revendication 15, dans lequel ledit doigt flexible (20) fait partie d'un organe actionneur (7) monté coulissant entre ledit corps interne (2) du distributeur et ledit réservoir (4), ledit corps interne (2) définissant une fenêtre (30) à travers laquelle ledit doigt flexible (20) coopère avec ledit élément de comptage (10), ladite fenêtre (30) comportant une butée de déplacement axial (31) pour ledit doigt flexible (20), de sorte qu'un déplacement axial supplémentaire entraîne une déformation élastique dudit doigt flexible (20).

17. Distributeur selon l'une quelconque des revendications 10 à 12, dans lequel le réservoir (4) est monté fixement dans le corps (1) du distributeur, ledit distributeur comportant un élément de distribution pour actionner ledit organe de distribution, lesdits moyens d'actionnement (20) dudit dispositif compteur de doses étant solidaire dudit élément de distribution.

18. Distributeur selon la revendication 17, dans lequel ledit élément de distribution est un capot pivotant entre une position de fermeture de l'orifice de distribution (5) et une position de distribution, l'organe de distribution (3) étant une pompe déclenchable par l'inhalation.

## Claims

1. A dose counter device for a fluid dispenser, said device comprising counting means (10, 11) actuated by actuating means (20) each time the dispenser is used, said device being **characterized in that** said counting means comprise at least one flexible rotary counting element (10) applied against and turning on a curved support surface (1, 2).

2. A device according to claim 1, in which said flexible counting element (10) is deformed each time it is actuated so as to remain in contact with said curved support surface (1, 2).

3. A device according to claim 1 or 2, in which said flexible counting element (10) is in the form of a disk, and is provided with a ring of serrations (15) or the like around its outer edge, which ring of serrations is suitable for co-operating with said actuating means (20).

4. A device according to any preceding claim, in which said counting means comprise two superposed flexible rotary counting elements (10, 11), said actuating means (20) co-operating with the first counting element (10), and coupling means (70, 80) being provided between the two counting elements (10, 11) for causing the second counting element (11) to turn, said coupling means (70, 80) being mounted to move between a non-coupling position and a coupling position.

5. A device according to claim 4, in which said counting elements (10, 11) turn about a common axis of rotation (60), said coupling means comprising a resilient tab (70) secured to or integral with one of the two counting elements (10, 11) and co-operating with a slot (80) provided in the other counting element (11, 10), said tab (70) being urged into the coupling position by cam means (90) each time the first coupling element (10) has turned through one full turn.

6. A device according to claim 4, in which said counting elements (10, 11) turn about mutually offset parallel axes of rotation (60, 61), said coupling means comprising a stud (70) that is secured to or integral with one of the two counting elements (10, 11), that co-operates with a projection (80) secured to or integral with the other counting element (11, 10), and that comes into the coupling position each time the first counting element (10) turns through one full turn.

7. A device according to any one of claims 4 to 6, in which the second counting element (11) is implemented in the form of a flexible disk or of an angular segment of flexible disk.

8. A device according to any one of claims 4 to 7, in which one of the two superposed flexible counting elements (10, 11) is provided with a window (100) for viewing the other counting element (11, 10) in part.

9. A device according to any one of claims 4 to 8, in which one of the two superposed flexible counting elements (10, 11) is organized to be transparent for the purpose of viewing the other counting element (11, 10).

10. A dispenser for dispensing a fluid, said dispenser comprising a fluid reservoir, a dispensing member (3), such as a pump or a valve, and a dispensing orifice (5), for selectively dispensing said fluid, said dispenser being **characterized in that** it further comprises a dose counter device according to any one of claims 1 to 9.

11. A dispenser according to claim 10, in which said at least one counting element (10) is disposed between two concentric substantially cylindrical surfaces (1, 2), the axis of rotation (60) of said at least one counting element (10) being substantially perpendicular to said substantially cylindrical surfaces (1, 2).

12. A dispenser according to claim 10 or 11, in which said at least one counting element (10) is disposed between the outer body (1) of the dispenser and an inner body (2) inside which the reservoir (4) is mounted.

13. A dispenser according to claim 12, in which said reservoir (4) is mounted to slide inside said inner body (2), said reservoir (4) co-operating with said actuating means (20) for actuating the dose counter device, so that, by moving, the reservoir (4) causes the counter to be actuated.

14. A dispenser according to claim 13, in which said actuating means (20) comprise an actuating finger (20) mounted to be moved with the reservoir (4) so as to cooperate with said counting element (10), said finger (20) being flexible, at least in the direction in which the reservoir (4) moves, so that it can adapt to accommodate various displacement strokes over which the reservoir is moved.

15. A dispenser according to claim 14, in which the dispensing member (3) is a metering valve adapted to dispensing a fluid with a propellant gas, the displacement stroke of the reservoir (4) corresponding to the actuating stroke of the valve member of the metering valve (3).

16. A dispenser according to claim 15, in which said flexible finger (20) is part of an actuator member (7) mounted to slide between said inner body (2) of the dispenser and said reservoir (4), said inner body (2) defining a window (30) through which said flexible finger (20) co-operates with said counting element (10), said window (30) being provided with an abutment (31) against which said flexible finger (20) comes when it moves axially, so that any additional axial movement causes said flexible finger (20) to be deformed elastically.

17. A dispenser according to any one of claims 10 to 12, in which the reservoir (4) is mounted to be fixed in the body (1) of the dispenser, said dispenser further comprising a dispensing element for actuating said dispensing member, said actuating means (20) for actuating said dose counter device being constrained to move with said dispensing element.

18. A dispenser according to claim 17, in which said dispensing element is a cover mounted to pivot between a position in which the dispensing orifice (5) is closed, and a dispensing position, the dispensing member (3) being a pump triggerable by inhaling.

## Patentansprüche

1. Dosen-Zählvorrichtung für eine Fluid-Abgabevorrichtung, die Zählmittel (10, 11) umfaßt, die durch Betätigungsmittel (20) bei jeder Verwendung der Abgabevorrichtung betätigt werden, **dadurch gekennzeichnet, daß** die Zählmittel wenigstens ein drehbares, flexibles Zählelement (10) umfassen, das an einer gekrümmten Tragoberfläche (1, 2) anliegt und sich auf dieser dreht.

2. Vorrichtung nach Anspruch 1, bei der das flexible Zählelement (10) sich bei jeder Betätigung verformt, um mit der gekrümmten Tragoberfläche (1, 2) in Berührung zu bleiben.

3. Vorrichtung nach Anspruch 1 oder 2, bei der das flexible Zählelement (10) die Form einer Scheibe besitzt und eine ringförmige Zahnung (15) oder dergleichen an seinem Außenrand aufweist, die mit besagten Betätigungsmitteln (20) zusammenwirken kann.

4. Vorrichtung nach einem der vorhergehenden Ansprüche, bei der die Zählmittel zwei drehbare, flexible, einander überlagerte Zählelemente (10, 11) umfassen, wobei die Betätigungsmittel (20) mit dem ersten Zählelement (10) zusammenwirken und Kopplungsmittel (70, 80) zwischen den beiden Zählelementen (10, 11) vorgesehen sind, um das zweite Zählelement (11) für eine Drehung anzutreiben, wobei die Kopplungsmittel (70, 80) zwischen einer Stellung, in der sie keine Verbindung herstellen, und einer Kopplungsstellung verschiebbar sind.

5. Vorrichtung nach Anspruch 4, bei der die Zählelemente (10, 11) sich um eine gemeinsame Drehachse (60) drehen, wobei die Kopplungsmittel eine elastische Zunge (70) umfassen, die fest mit einem der beiden Zählelemente (10, 11) verbunden ist und mit einem Schlitz (80) zusammenwirkt, der in dem anderen Zählelement (11, 10) vorgesehen ist, wobei diese Zunge (70) bei jeder vervollständigten Umdrehung des ersten Zählelementes (10) durch Nockeneinrichtungen (90) in die Kopplungsstellung vorgespannt wird.

6. Vorrichtung nach Anspruch 4, bei der die Zählelemente (10, 11) sich um gegeneinander versetzte, zueinander parallele Rotationsachsen (60, 61) drehen, wobei die Kopplungsmittel einen Nocken (70) umfassen, der mit einem der beiden Zählelemente (10, 11) fest verbunden ist und mit einem Vorsprung (80) zusammenwirkt, der mit dem anderen Zählelement (11, 10) fest verbunden ist und bei jeder vervollständigten Umdrehung des ersten Zählelementes (10) in eine Kopplungsposition gelangt.

7. Vorrichtung nach einem der Ansprüche 4 bis 6, bei der das zweite Zählelement (11) in Form einer flexiblen Scheibe oder eines Winkelsegments einer flexiblen Scheibe ausgeführt ist.

8. Vorrichtung nach einem der Ansprüche 4 bis 7, bei der eines der beiden flexiblen, einander überlagerten Zählelemente (10, 11) ein Fenster (100) umfaßt, um das andere Zählelement (11, 10) teilweise sichtbar zu machen.

9. Vorrichtung nach einem der Ansprüche 4 bis 8, bei der eines der beiden flexiblen, einander überlagerten Zählelemente (10, 11) in transparenter Weise ausgeführt ist, um das andere Zählelement (11, 10) sichtbar zu machen.

10. Abgabevorrichtung für ein Fluid, die einen Produktbehälter, ein Abgabeorgan (3), wie zum Beispiel eine Pumpe oder ein Ventil, und eine Abgabeöffnung (5) umfaßt, um wahlweise das Produkt abzugeben, **dadurch gekennzeichnet, daß** die Abgabevorrichtung eine Dosen-Zählvörrichtung nach einem der Ansprüche 1 bis 9 umfaßt.

11. Abgabevorrichtung nach Anspruch 10, bei der das wenigstens eine Zählelement (10) zwischen zwei in etwa zylindrischen, konzentrischen Oberflächen (1, 2) angeordnet ist, wobei die Rotationsachse (60) des wenigstens einen Zählelementes (10) ungefähr senkrecht zu den in etwa zylindrischen Oberflächen (1, 2) verläuft.

12. Abgabevorrichtung nach Anspruch 10 oder 11, bei der das wenigstens eine Zählelement (10) zwischen dem Außenkörper (1) der Abgabevorrichtung und einem Innenkörper (2) angeordnet ist, in dessen Inneren der Behälter (4) montiert ist.

13. Abgabevorrichtung nach Anspruch 12, bei der der Behälter (4) im Inneren des Innenkörpers (2) gleitend montiert ist, wobei der Behälter (4) mit den Betätigungsmitteln (20) der Dosen-Zählvorrichtung derart zusammenwirkt, daß eine Verschiebung des Behälters (4) eine Betätigung der Zähleinrichtung bewirkt.

14. Abgabevorrichtung nach Anspruch 13, bei der die Betätigungsmittel (20) einen Betätigungszapfen (20) umfassen, der zusammen mit dem Behälter (4) verschiebbar ist, um mit dem Zählelement (10) zusammenzuwirken, wobei der Zapfen (20) zumindest in der Verschieberichtung des Behälters (4) derart flexibel ist, daß er sich an die variablen Verschiebungshübe des Behälters anpassen kann.

15. Abgabevorrichtung nach Anspruch 14 bei der das Abgabeorgan (3) ein Dosierventil ist, das in der Lage ist, ein Produkt mit einem Treibgas abzugeben, wobei der Verschiebungshub des Behälters (4) dem Betätigungshub des Ventils des Dosierventils (3) entspricht.

16. Abgabevorrichtung nach Anspruch 15, bei der der flexible Zapfen (20) Teil eines Betätigungsorgans (7) bildet, das in gleitender Weise zwischen dem Innenkörper (2) der Abgabevorrichtung und dem Behälter (4) montiert ist, wobei der Innenkörper (2) ein Fenster (30) aufweist, durch das hindurch der flexible Zapfen (20) mit dem Zählelement (10) zusammenwirkt, wobei das Fenster (30) einen axialen Verschiebungsanschlag (31) für den flexiblen Zapfen (20) derart umfaßt, daß eine ergänzende axiale Verschiebung eine elastische Verformung des flexiblen Zapfens (20) bewirkt.

17. Abgabevorrichtung nach einem der Ansprüche 10 bis 12, bei der der Behälter (4) fest im Körper (1) der Abgabevorrichtung montiert ist, wobei die Abgabevorrichtung ein Abgabeelement umfaßt, das dazu dient, das Abgabeorgan zu betätigen, wobei die Betätigungsmittel (20) der Dosen-Zählvorrichtung fest mit dem Abgabeelement verbunden sind.

18. Abgabevorrichtung nach Anspruch 17, bei der das Abgabeelement eine Kappe ist, die zwischen einer Verschlußstellung für die Abgabeöffnung (5) und einer Abgabestellung verschwenkbar ist, wobei das Abgabeorgan (3) eine durch Inhalation auslösbare Pumpe ist.
